# EUROPEAN PATENT SPECIFICATION

(11) **EP 0 958 268 B1**
(45) Date of publication and mention of the grant of the patent: **04.05.2005**
(21) Application number: 98951986.3
(22) Date of filing: 30.09.1998
(51) Int. Cl.: C07G 17/00, C07C 39/12

(54) **SPHERICAL MOLECULAR ASSEMBLY**
KUGELFÖRMIGE MOLEKÜLGRUPPIERUNG
ENSEMBLE MOLECULAIRE SPHERIQUE

(30) Priority: 01.10.1997 US 60591 P; 27.03.1998 US 79616 P
(43) Date of publication of application: 24.11.1999
(73) Proprietor: Macgillivray, Leonard R., Columbia, MO 65211 (US); Atwood, Jerry L., Columbia, MO 65211 (US)
(72) Inventor: Macgillivray, Leonard R., Columbia, MO 65211 (US); Atwood, Jerry L., Columbia, MO 65211 (US)
(74) Representative: D'Agostini, Giovanni, Dr.
(86) International application number: PCT/US1998/020389
(87) International publication number: WO 1999/016734

(56) References cited:
- CHAPMAN et al., "Templation and Encapsulation in Supramolecular Chemistry", TETRAHEDRON, 24 November 1997, Vol. 53, No. 47, pages 15911-15945, XP002915653
- BROUWER et al., "Solid-State NMR and Diffraction Studies of a Tunable p-tert-Butylcalix(4)arene. Guest Structure", J. AM. CHEM. SOC., 11 June 1997, Vol. 119, No. 23, pages 5405-5412, XP002915654
- BOTT et al., "Intercalation of Cationic, Anionic and Molecular Species by Organic Hosts Preparation and Crystal Structure of [NH4]6[calix[4]arenesulfonate][MeOSO3]. (H2O)2", J. AM. CHEM. SOC., 1988, Vol. 110, pages 610-611, XP002915655

## Description

### BACKGROUND OF THE INVENTION

### Field of the Invention

The present invention generally relates to the preparation of molecular or ionic supramolecular assemblies having a substantially enclosed volume and a framework structure that mimics a Platonic or Archimedean solid.

### Related Art

Spontaneous self-assembly processes that lead to discrete spherical arrays are common in Nature. Indeed, spherical viruses (Caspar et al. , D. & Klug, A. Physical principles in the construction of regular viruses. Cold Spring Harb. Symp. Quant. Biol. 27, 1-24 (1962)) (e.g. hepatitis B) and fullerenes (Kroto et al., H. W., Heath, J. R., O'Brien, S. C., Curl, R. F., Smalley, R. E. C₆₀: Buckminsterfullerene. Nature 318, 162-163 (1985)) (e.g. C₆₀) are well known examples in which noncovalent and covalent forces, respectively, direct the assembly of smaller sub-units (or synthons) into larger superstructures. A common feature of these shell-like architectures is their ability to encapsulate neutral and/or charged guests whose size, shape, and chemical exteriors complement their inner surfaces. (Casjens, S. Nucleic acid packaging by viruses, in Virus Structure and Assembly (ed. Casjens, S.) 75-147, Jones and Bartlett, Boston, 1995; Schwarz et al., H., Weiske, T., Bohme, D. K., Hrusdak, J. Exo- and endohedral fullerene complexes in the gas phase, in Buckminsterfullerenes, eds. Billups, W. E. & Ciufolini, M. A., 257-283, VCH, New York, 1993). Their interiors can often be regarded as unique phases of matter (Sherman et al. , J. C. & Cram, D. J. Carcerand interiors provide a new phase of matter. J. Am. Chem. Soc. 111, 4527-4528 (1989)) capable of controlling the flow of reactants, transients, and products and catalyzing reactions of both chemical and biological relevance. Such properties have inspired the recent emergence of mono- and supramolecular dimeric molecular capsules (Sherman et al.; Garel et al., L, tasta, J.-P., Collet, A. Complexation of methane and chlorofluorocarbons by cryptophane-A in organic solution. Angew. Chem. Int. Ed Engl. 32, 1169-1171 (1993); Timmerman et at., P., Verboom, W., van Veggel, F. C., van Duynhoven, J. P., Reinhoudt, D. N. A novel type of stereoisomerism in calix[4]arene-based carceplexes. Angew. Chem. Int.. Ed Engl. 33, 2345-2348 (1994); (Kang et at. , J. & Rebek, J. Jr. Acceleration of a Diels-Alder reaction by a self-assembled molecular capsule. Nature 385, 50-52 (1997); Shimizu et at. , K. D., Rebek, J. Jr. Synthesis and assembly of self-complementary calix[4]arenes. Proc. Natl. Acad. Sci. USA. 92, 12403-12407 (1995)), many of which have been based upon the head-to-head alignment of bowl-shaped polyaromatic macrocycles such as calix[4]arenes. Despite the synthesis of these pseudo-sphrical capsules, however, structural mimicry of frameworks akin to viruses and fullerenes, which are based upon the self-assembly of n > 3 sub-units where surface curvature is supplied by edge sharing of regular polygons and/or polygons that exhibit quasi-equivalence, has remained elusive and promises to bear relevance in a number of areas including biology, chemistry, and materials science

### SUMMARY OF THE INVENTION

The scope is reached with the features of main claim, namely by a substantially spherical supramolecular assembly having a substantially enclosed volume and a framework structure that mimics a Platonic or Archimedean solid, the supramolecular assembly comprising more than four synthons held together by a combination of carbon-based covalent, non-covalent, and/or coordinate covalent bonds, with at least two of the bonds being selected from the group consisting of noncovalent bonds and coordinate covalent bonds, wherein the synthons comprise water molecules and calixresorcinarenes of the formula: 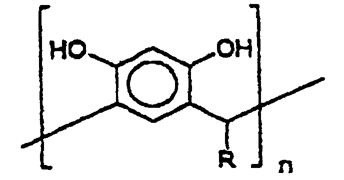 wherein n is an integer within the range of 4-8 and the R groups are independently selected from the group consisting of hydrocarbon, heterosubstituted hydrocarbon, and heteroaryl groups.

Among the objects of the invention, therefore, is the provision of a supramolecular assembly having a substantially enclosed volume and a framework structure that mimics a Platonic or Archimedean solid.

Briefly, therefore, the present invention is directed to a substantially spherical supramolecular assembly having a substantially enclosed volume and framework structure that mimics a Platonic or Archimedean solid. The supramolecular assembly comprises more then four synthons held together by a combination of carbon-based covalent, non-covalent, and/or coordinate covalvalent bonds provided at least two of the bonds are selected from non-covalent and coordinate covalent bonds.

The present invention is further directed to a process for preparing a substantially spherical supramolecular assembly having a substantially enclosed volume and a framework structure that mimics a Platonic or Archimedean solid. (The disclosure contained in MacGillivray et al. (Nature 389 469 (1997))). The process comprises combining more then four synthons in a solvent in a proportion with the stoichiometry being based upon the following table, in which "c" represents th num (so that, e.g., "fe" is the number of three-sided faces):

| NAME | c | e | f3 | f4 | f5 | f6 | f8 | F10 |
|---|---|---|---|---|---|---|---|---|
| cube | 8 | 12 | - | 6 | - | - | - | - |
| octahedron | 6 | 12 | 8 | - | - | - | - | - |
| dodecahedron | 20 | 20 | - | - | 12 | - | - | - |
| icosohedron | 18 | 24 | 20 | - | - | - | - | - |
| truncated tetrahedron | 12 | 18 | 4 | - | - | 4 | - | - |
| truncated cube | 24 | 36 | 8 | - | - | - | 6 | - |
| truncated octahedron | 24 | 36 | - | 6 | - | 8 | - | - |
| cuboctahedron | 12 | 24 | 8 | 6 | - | - | - | - |
| small | 24 | 48 | 8 | 18 | - | - | - | - |
| rhombicuboctahedron great | 48 | 72 | - | 12 | - | 8 | 6 | - |
| rhombicuboctahedron snub cube | 24 | 60 | 32 | 6 | - | - | - | - |
| truncated dodecahedron | 60 | 90 | 20 | - | - | - | - | 12 |
| truncated icosahedron | 60 | 90 | - | - | 12 | 20 | - | - |
| icosidodecahedron | 30 | 60 | 20 | - | 12 | - | - | - |
| small | 60 | 120 | 20 | 30 | 12 | - | - | |
| rhombicosidodecahedron great | 120 | 180 | - | 30 | - | 20 | - | 12 |
| rhombicosidodecahedron snub dodecahedron | 60 | 150 | 80 | - | 12 | - | - | - |

Other objects and features of this invention will be apparent and still others will be pointed out hereinafter.

### BRIEF DESCRIPTION OF THE DRAWINGS

Fig. 1. The structure formed from 6 C-methylcalix[4]resorcinarenes (see 2a defined in Fig. 2 below) 8 H2O, hereinafter referred to as "supramolecular assembly 1a" (a) cross-sectional view; (b) space filling views along the crystallographic 4-fold rotation axis; (c) 3-fold rotation axis; (d) 2-fold rotation axis; (e) a cut-away view along the 3-fold rotation axis, (f) solid state packing,
where the shaded dark grey spheres also represent supramolecular assembly la (solvent molecules have been omitted for clarity);

Fig. 2. Molecular structures of calix[4Iresorcinarene (2); C-methylcalix[4]resorcinarene (2a); C-undecylcalix[4] resorcinarene (2b).

Fig. 3. The snub cube - one of the thirteen Archimedean solids. The square faces correspond to the calixarenes; the eight shaded triangles that adjoin three squares correspond to the water molecules of supramolecular assembly la.

Fig. 4. The hydrogen bond pattern of supramolecular assembly la: (a) the five hydrogen bonds that comprise each "edge' of the water cuboid, (b) D_{2d} symmetry representation of the water cuboid, (c) the three calixarene conformers that are associated with the faces of the water cuboid.

Fig. 5. 500 MHz ¹H NMR spectrum of C-undecylcalix[4Iresorcinarene in benzene-d6.

### DETAILED DESCRIPTION OF THE INVENTION

### Definitions

As used herein, the term "synthon" shall mean a molecular or ionic building block capable of interacting with itself or other molecular and/or ionic building blocks by way of non-covalent and/or coordinate covalent bonds.

The term "sub-unit" shall mean regular polygons and/or polygons that exhibit quasi-equivalence.

The term "quasi-equivalence" shall mean the relaxation of symmetry of regular polygons.

The term "Ar" means aryl; "Ph" means phenyl; "Me" means methyl, and "R" means lower alkyl unless otherwise defined.

The term "substantially enclosed volume", as used herein means a volume located within the interior region of an supramolecular assembly, that may or may not be accessible by ions, molecules, or combinations of ions and/or molecules.

The term "substantially spherical" as used herein means an supramolecular assembly that possesses a surface that approximates the shape of a sphere such that its framework structure mimics a Platonic or Archimedean solid.

The term "Platonic solid" as used herein includes tetrahedrons, cubes, octahedron, dodecahedrons, and icosahedrons, and the term 'Archimedean solid" as used herein includes truncated tetrahedrons, truncated cubes, truncated octahedrons, cuboctahedrons, small rhombicuboctahedrons, great rhomicuboctahedrons, snub cubes, truncated dodecahedrons, truncated icosahedrons, icosidodecahedrons, small rhombicosidodecahedrons, great 15 rhombicosidodecahedrons, and snub dodecahedrons. These solids have the number of comers ("c"), edges ("e"), and faces (" 13, f4, 15, f6, f8, and f10") as indicated in the following table wherein "13" means a three-sided face, "14" means a four-sided face, etc.:

| NAME | c | e | f3 | f4 | f5 | f6 | f8 | F10 |
|---|---|---|---|---|---|---|---|---|
| cube | 8 | 12 | - | 6 | - | - | - | - |
| octahedron | 6 | 12 | 8 | - | - | - | - | - |
| dodecahedron | 20 | 20 | - | - | 12 | - | - | - |
| icosohedron | 18 | 24 | 20 | - | - | - | - | - |
| truncated tetrahedron | 12 | 18 | 4 | - | - | 4 | - | - |
| truncated cube | 24 | 36 | 8 | - | - | - | 6 | - |
| truncated octahedron | 24 | 36 | - | 6 | - | 8 | - | - |
| cuboctahedron | 12 | 24 | 8 | 6 | - | - | - | - |
| small | 24 | 48 | 8 | 18 | - | - | - | - |
| rhombicuboctahedron great | 48 | 72 | - | 12 | - | 8 | 6 | - |
| rhombicuboctahedron snub cube | 24 | 60 | 32 | 6 | - | - | - | - |
| truncated dodecahedron | 60 | 90 | 20 | - | - | - | - | 12 |
| truncated icosahedron | 60 | 90 | - | - | 12 | 20 | - | - |
| icosidodecahedron | 30 | 60 | 20 | - | 12 | - | - | - |
| small | 60 | 120 | 20 | 30 | 12 | - | - | - |
| rhombicosidodecahedron great | 120 | 180 | - | 30 | - | 20 | - | 12 |
| rhombicosidodecahedron snub dodecahedron | 60 | 150 | 80 | - | 12 | - | - | - |

The "hydrocarbon" moieties described herein (other than the hydrocarbon solvents) are organic radicals or substituents consisting exclusively of the elements carbon and hydrogen. These moieties include alkyl, alkenyl, alkynyl, and aryl moieties. These moieties also include alkyl, alkenyl, alkynyl, and aryl moieties substituted with other aliphatic or cyclic hydrocarbon groups, such as alkaryl, alkenaryl and alkynaryl. Preferably, these moieties comprise 1 to 20 carbon atoms.

The alkyl groups described herein are preferably lower alkyls containing from one to 20 carbon atoms in the principal chain and up to 40 carbon atoms. They may be straight or branched chain and include methyl, ethyl, propyl, isopropyl, butyl, hexyl, and the like. They may be substituted with aliphatic or other hydrocarbon radicals.

The alkenyl groups described herein are preferably lower alkenyl containing from two to 20 carbon atoms in the principal chain and up to 40 carbon atoms. They may be straight or branched chain and include ethenyl, propenyl, isopropenyl, butenyl, isobutenyl, hexenyl, eicosenyl, and the like. They may be substituted with aliphatic or other hydrocarbon radicals.

The alkynyl groups described herein are preferably lower alkynyl containing from two to 20 carbon atoms in the principal chain and up to 40 carbon atoms. They may be straight or branched chain and include ethynyl, propynyl, butynyl, isobutynyl, hexynyl, eicosynyl, and the like. They may be substituted with aliphatic or other hydrocarbon radicals.

The aryl moieties described herein have one to three rings, contain from 6 to 20 carbon atoms and include phenyl, biphenyl, naphthyl, anthracenyl, fluorene, acenaphthylene, and the like. They may be substituted with hydrocarbon radicals, heterosubstituted hydrocarbon, or hetero-atom containing substituents, with the hetero-atoms being selected from the group consisting of nitrogen, oxygen, silicon, phosphorous, boron, sulfur, and halogens. These substituents include lower alkoxy such as methoxy, ethoxy, butoxy; halogen such as chloro or fluoro; ethers; acetals; ketals; esters; heteroaryl such as furyl or thienyl; alkanoxy; hydroxy; protected hydroxy; acyl; acyloxy; nitro; amino; and amido. Phenyl is the more preferred aryl.

The heteroaryl moieties described are heterocyclic compounds or radicals that are analogous to aromatic compounds or radicals and that contain a total of 5 to 20 atoms, usually 5 or 6 ring atoms, and at least one atom other than carbon, such as furyl, thienyl, pyridyl and the like. The heteroaryl moieties may be substituted with hydrocarbon, heterosubstituted hydrocarbon or hetero-atom containing substituents with the hetero-atoms being selected from the group consisting of nitrogen, oxygen, silicon, phosphorous, boron, sulfur, and halogens. These substituents include lower alkoxy such as methoxy, ethoxy, butoxy; halogen such as chloro or fluoro; ethers; acetals; ketals; esters; heteroaryl such as furyl or thienyl; alkanoxy; hydroxy; protected hydroxy; acyl; acyloxy; nitro; amino; and amido.

The heterosubstituted hydrocarbon moieties described herein are hydrocarbon moieties that are substituted with at least one atom other than carbon, including moieties in which a carbon chain atom is substituted with a hetero atom such as nitrogen, oxygen, silicon, phosphorous, boron, sulfur, or a halogen atom. These substituents include lower alkoxy such as methoxy, ethoxy, butoxy; halogen such as chioro or fluoro; ethers; acetals; ketals; esters; heteroaryl such as furyl or thienyl; alkanoxy; hydroxy; protected hydroxy; acyl; acyloxy; nitro; amino; and amido.

### Description

The present invention concerns a substantially spherical supramolecular assembly having a substantially enclosed volume and a framework structure that mimics a Platonic or Archimedean solid, the supramolecular assembly comprising more than four synthons held together by a combination of carbon-based covalent, non-covalent, and/or coordinate covalent bonds, with at least two of the bonds being selected from the group consisting of noncovalent bonds and coordinate covalent bonds, wherein the synthons comprise water molecules and calixresorcinarenes of the formula: 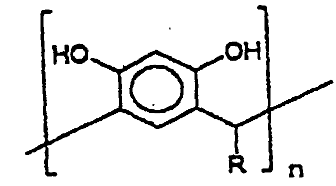 wherein n is an integer within the range of 4-8 and the R groups are Independently selected from the group consisting of hydrocarbon, heterosubstituted hydrocarbon, and heteroaryl groups.

Calixresorcinarenes, with calix[4]resorcinarene being preferred and corresponding to the structure 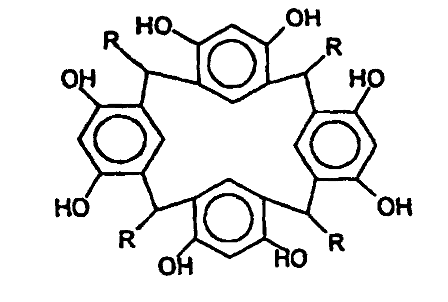 wherein R is hydrocarbon, heterosubstituted hydrocarbon, or heteroaryl; In general, there are two types of non-covalent interactions or bonds that may be used to bond the synthons: non-directional and directional. Although directional bonds are preferred for the construction of closed surface substantially spherical assemblies, non- directional bonds (electrostatic type) may be useful. The possible combinations are ion-ion, ion-dipole, ion-induced dipole, dipole-dipole, dipole/induced dipole and induced dipole-induced dipole. See, e.g., "Physical Methods in Inorganic Chemistry" by R. S. Drago, Reinhold Publishing Corp., New York, 1965, p.68ff.). Also included in this classification are n-stacking interactions. Van der Waals interactions may also be employed.

Of the various types of directional bonds, hydrogen bonds are preferred and the most preferred of these are O-H...O, N-H...O, O-H...N, F-H...F, F-H...0, 0-H... F, F-H... N, and N-H... F. Other hydrogen bonds such as C-H...F, C-H...O, C-H...N, C-H...Cl, C-H...S, C-H...Br, C-H...I, O-H...Cl, O-H...Br, OH... I, N-H...Cl, N-H...Br, N-H...I, S-H...O, S-H... N, and S-H...F may, nevertheless, be used.

In the preferred supramolecular assembly utilizing hydrogen bonds, the synthons must be hydrogen bond donors (e.g., N-H), hydrogen bond acceptors (e.g., 0), or an appropriate combination of hydrogen bond donors and hydrogen bond acceptors (e.g., 0) and the other synthons must be hydrogen bond acceptors, hydrogen bond donors (e.g., N-H), or an appropriate combination of hydrogen bond donors and hydrogen bond acceptors.

The choice of synthons involves the selection of synthons that provide a means by which the edge-sharing faces of the Platonic or Archimedean solids are bonded together.

When a combination of synthons with the appropriate hydrogen bonding ability has been selected, consideration is preferably given to steric interactions. If the van der Waals volume of an atom or atoms on one synthon is required to occupy the same space as the van der Waals volume of an atom or atoms on another synthon, the combination is inoperative.

The molecular or ionic supramolecular assembly of the present invention is prepared by combining the synthons in the required stoichiometric ratio in a solvent. Time and temperature do not provide limitations on the process. However, the assemblies may be affected by extraordinary high temperature. The crystallization is preferably carried out at between about 15 and about 25°C.

The solvent in which the synthons are mixed does not appear to be narrowly critical. The synthons may be combined in one or more of the following solvents: hydrocarbon based such as hexane, heptane, benzene, or toluene; halogenated hydrocarbons such as chloroform, carbon tetrachloride, or methylene chloride; nitromethane, nitrobenzene, acetone, tetrahydrofuran, MIBK, DMSO, DMF, propylene carbonate, acetonitrile, methanol, ethanol, propanol, isopropanol, pyridine, and water. The hydrogen-bonding ability of the solvent, however, must not interfere with that of the supramolecular assembly.

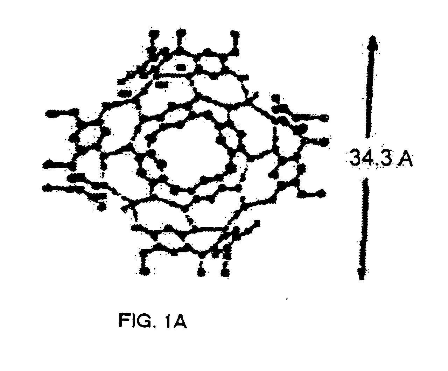

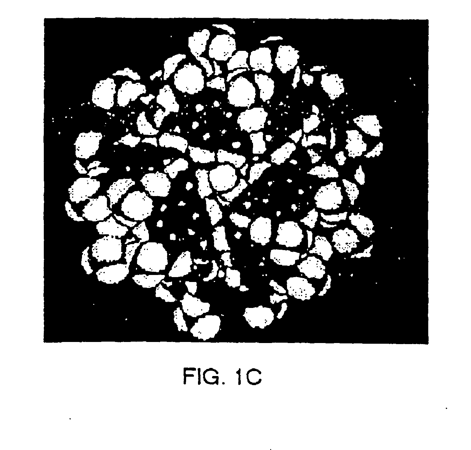

In accordance with the present invention, a chiral supramolecular assembly 1a that consists of six C-methylcalix[4]resorcinarenes and eight water molecules and is held together by 60 hydrogen bonds is illustrated in Fig. 1a. This supramolecular assembly has been found to maintain its structure in apolar media and encapsulate guest species within a well-defined cavity that possesses an internal volume of 1375 A³. Single crystal X-ray analysis has revealed the topology of supramolecular assembly 1a to resemble a spherical virus and conform to the structure of a snub cube, one of the thirteen Archimedean solids (Wenninger, *Polyhedron Models,* Cambridge University Press, New York, 1971).

Co-crystallization studies of C-methylcalix[4]resorcinarene with hydrogen bond acceptors in aromatic solvents have revealed the ability of C-methylcalix[4]resorcinarene to self-assemble as a spherical hexamer, along with adventitious water molecules to form supramolecular assembly 1 a. In a preferred method, the water is added in a stoichiometric amount. Solution studies have revealed the ability of C-undecylcalix[4]resorcinarene to maintain a structure analogous to that of supramolecular assembly 1 a in apolar organic solvents.

Addition of C-methylcalix[4]resorcinarene (0.015 g) to a boiling aliquot of neat nitrobenzene (5mL) followed by cooling to room temperature yielded light yellow cubic crystals suitable for X-ray analysis. This formulation of this compound was confirmed by single-crystal X-ray diffraction and ¹H NMR spectroscopy.

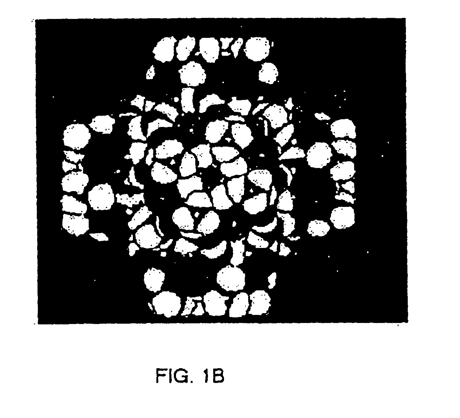

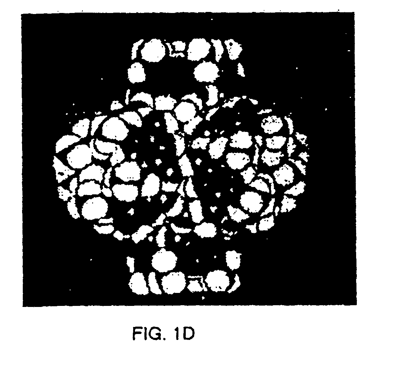

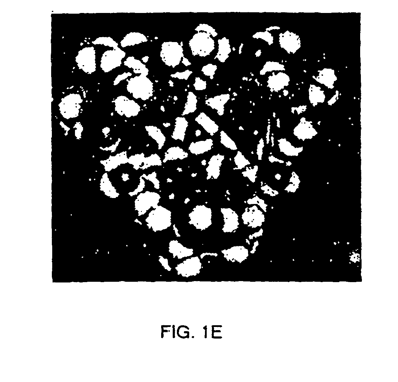

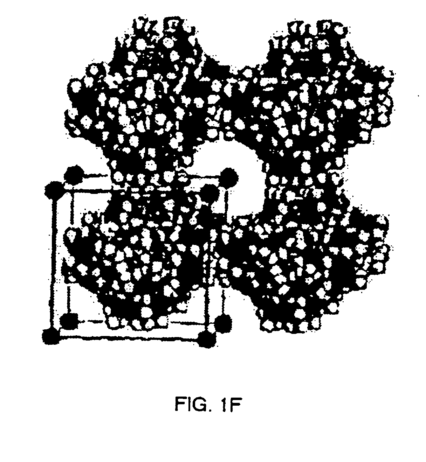

A cross sectional view of the X-crystal structure of supramolecular assembly 1 a is show in Fig. 1. The supramolecular assembly consists of six molecules of C-methylcalix[4]resorcinarene and eight molecules of water that have assembled, via 60 0-H...O hydrogen bonds, to form a shell-like cubic spheroid. The calixarenes, each of which lies around a four-fold axis, point their hydroxyl groups along the periphery of supramolecular assembly 1a and form two hydrogen bonds to two neighboring calixarenes while the water molecules, each of which lies on a threefold axis, are embedded along the surface of supramolecular assembly 1a such that they lie on the vertices of a cube (edge length = 9.00 A) and participate in three hydrogen bonds with three different calixarenes. Notably, each calixarene also exhibits four intramolecular hydrogen bonds, one at each of its comers, which impart stability to its bowl-like conformation. As a result, as shown in Fig. 1 b-d, supramolecular assembly 1a possesses 4 3 2 symmetry, ignoring all hydroxyl hydrogen atoms, and a well-defined central cavity with a maximum diameter of 17.7 A and an internal volume of 1375 A³ , as illustrated in Fig. 1e. Indeed, the cavity of supramolecular assembly 1a is vast, being more than 4.5 times larger than the cavity of the largest molecular capsule reported to date (ca. 300 A³). Interestingly, the calixarenes of supramolecular assembly 1a are twisted by 23° with respect to the faccesof the water "cuboid" which, as a consequence, makes supramolecular assembly 1a chiral.

Supramolecular assembly 1a self-assembles in the solid state such that neighboring spheroids fall on their four-fold axes and their methyl groups lie staggered owing to the twisting displayed by the macrocycles, as shown in Fig. 1f. This gives rise to an interpenetrating body-centered cubic lattice that exhibits interstices within supramolecular assembly 1a that are occupied by disordered water and nitrobenzene molecules. Despite being able to locate guest species within the interior of supramolecular assembly 1a (i.e. electron density maxima), it is not possible to determine their identity from the X-ray experiment presumably due to the high symmetry imposed by supramolecular assembly 1a and high thermal motion within the cavity.

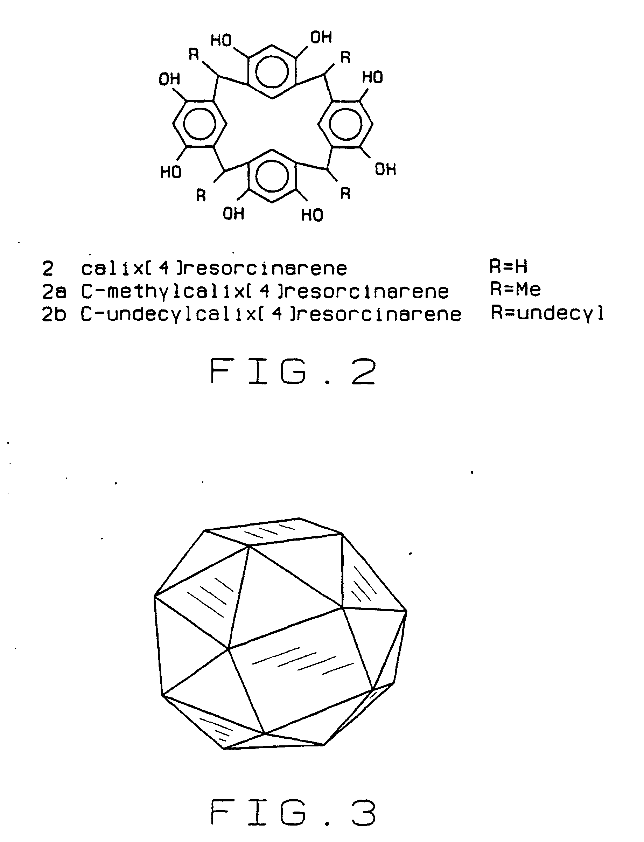

Polyhedron models revealed the structure of supramolecular assembly 1a to conform to a snub cube (Fig 3), one of the thirteen Archimedean solids, in which the vertices of the square faces correspond to the comers of the calixarenes and the centroids of the eight triangles that adjoin three squares correspond to the eight water molecules. It is clear to us that calixarenes and water molecules are not essential for the formation of the chemical version of the snub cube. Any molecules or ions with square and triangular symmetry, respectively, that can be bonded together by hydrogen bonds or other interactions will also form the chemical version of the snub cube. Indeed, the ability of six calixarenes and eight water molecules to self-assemble to form supramolecular assembly 1a is reminiscent of spherical viruses in which identical copies of proteins self-assemble, via noncovalent forces, to form viral capsids having icosahedral cubic symmetry and a shell-like enclosure. In fact, supramolecular assembly la is the first example of a closed surface supramolecular spheroid that possesses n > 3 sub-units and, owing to the fit exhibited by its components, possesses a topology that agrees with the theory of virus shell structure, which states that octahedral systems must contain 24 asymmetric units and possess 4 3 2 symmetry. Moreover, such observations illustrate that in order to design related hosts, one must consider the limited number of possibilities available in space for such frameworks, those being the five Platonic (regular) solids and the thirteen Archimedean (semiregular) solids. The ramifications of these observations are important since they suggest that appropriately sized, shaped, and functionalized components may be selected, a priori, to design similar spherical hosts in a similar way to the self- supramolecular assembly exhibited by supramolecular assembly 1a.

The hydrogen bond pattern that holds supramolecular assembly 1a together is complex.

Although the hydroxyl hydrogen atoms were not locateable, it is possible to deduce the pattern knowing the positions and numbers of hydrogen bond donors and acceptors. In principle, 64 hydrogen bond donors and 112 hydrogen bond acceptor sites are available to form supramolecular assembly 1a: eight donors and sixteen acceptor sites from each calixarene and two donors and two acceptor sites from each water molecule. There are, however, two restrictions that arise from the positions of these sites that require the number of hydrogen bonds that define supramolecular assembly 1a to equal 60 which, as a result, cause four hydroxyl hydrogen atoms to point away from its surface. The first restriction lies in the positions of the eight water molecules. Their location on the three fold axes limits the number of hydrogen bonds in which the water molecules can participate that contributes to supramolecular assembly 1 a to 24; in other words, each water molecule is capable of participating in only three hydrogen bonds along the surface of supramolecular assembly la and is therefore unable to use its two hydrogen bond donors and two hydrogen bond acceptor sites, simultaneously, to form supramolecular assembly 1a. The second restriction arises from the presence of the four intramolecular hydrogen bonds at the comers of the calixarenes. In particular, these interactions force four hydroxyl groups to point their hydrogen atoms above each macrocycle which, as a consequence, makes each calixarene a quadruple hydrogen bond donor. (MacGillivray et al. J. Am. Chem. Soc., 119 6931 (1997).) Since each calixarene uses two of these hydrogen atoms to form hydrogen bonds to two calixarenes, this leaves two hydroxyl hydrogen atoms per calixarene which are, subsequently, used to form 12 hydrogen bonds to the water molecules.

Consequently, the water molecules must use 12 of their hydrogen bond acceptor sites to form these hydrogen bonds and, owing to their position on the three fold axes, must position four hydrogen atoms, each from a different water molecule, away from the surface of supramolecular assembly 1a. To complete the hydrogen bond array, the water molecules use 12 hydrogen atoms to form 12 hydrogen bonds to 12 hydrogen bond acceptor sites located on the calixarenes.

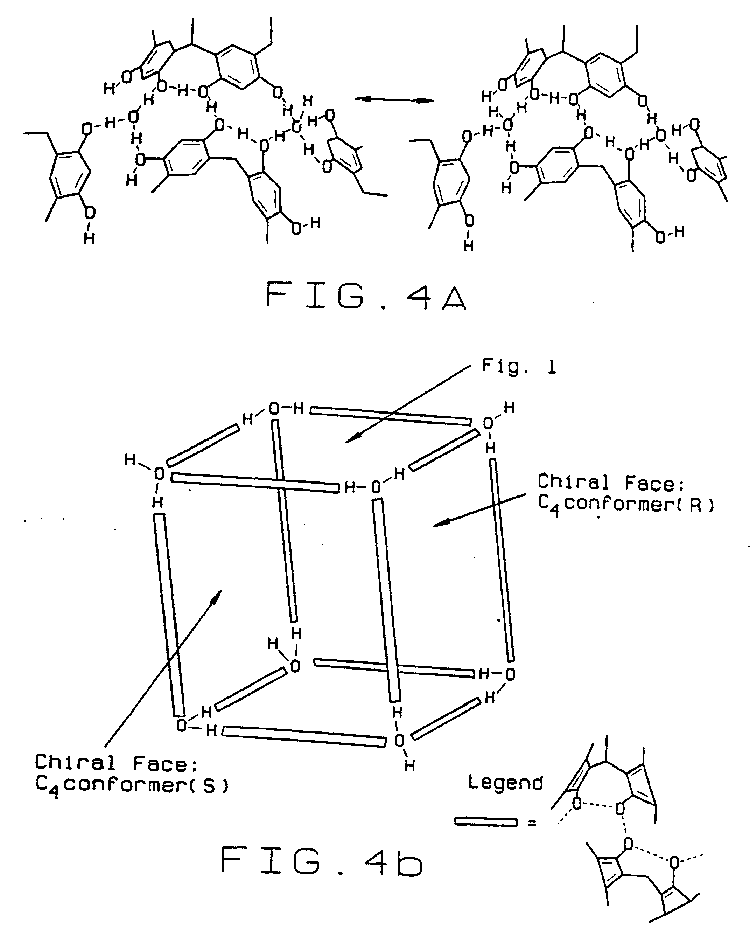

A closer inspection of the hydrogen bond pattern of supramolecular assembly 1 a reveals that each "edge" of the water cuboid consists of a polar chain of five cooperative hydrogen bonds, as shown in Fig. 4a.

As a result, each edge is capable of changing the sense of its direction via interconversion of two water molecules from a hydrogen bond donor to a hydrogen bond acceptor and vice versa.

Furthermore, since four water molecules point four hydrogen atoms away from the surface of supramolecular assembly 1 a, the edges of the cuboid are capable of undergoing such proton rearrangements simultaneously, which, in effect, makes supramolecular assembly 1a a spherical proton pump able to channel the four "dangling" protons to the eight comers of the water cuboid.

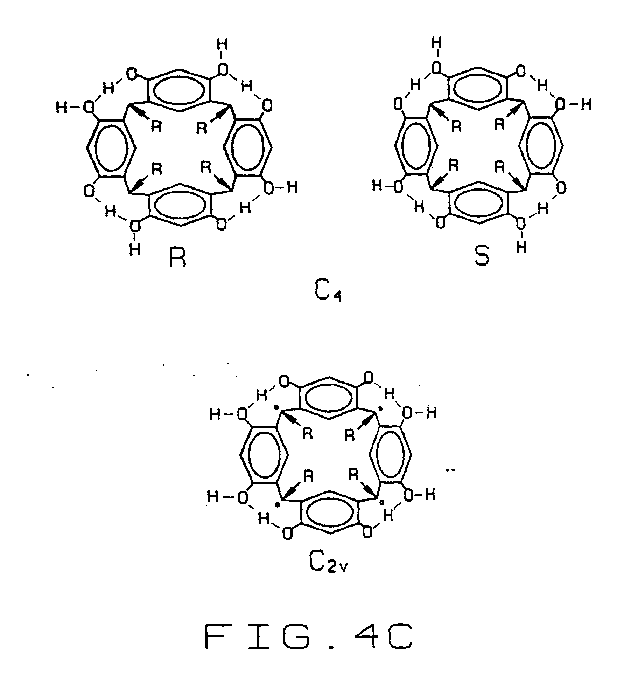

Although each of the dangling protons may be placed at any one of the eight comers, it is most convenient, for symmetry reasons, to place them at the vertices of a tetrahedron such that the water cuboid, ignoring the calixarenes, possesses D_{2d} symmetry, as shown in Fig. 4b. (Wales et al. J. Chem. Phys. 98, 7257-7268 (1993)). This arrangement furnishes the cuboid with four chiral faces and two achiral faces which, upon completing the five hydrogen bonds along each edge, gives rise to three distinct calixarenes, two of which are chiral (C₄) and one of which is achiral (C_{2V}), as shown in Fig. 4c. Notably, the chiral calixarenes are associated with the chiral faces such that their handedness match while the achiral calixarenes are associated with the achiral faces. As a consequence of this arrangement, supramolecular assembly 1a possesses D₂ symmetry.

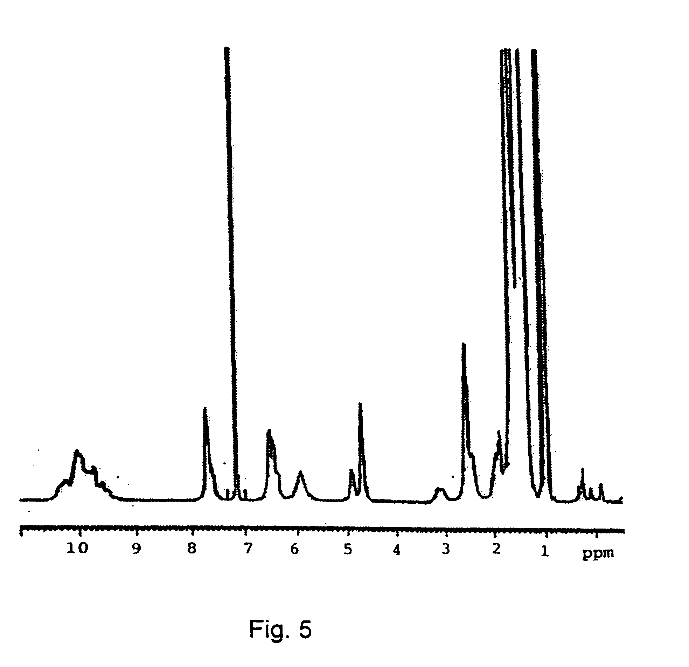

Evidence supporting the ability of C-undecylcalix[4]resorcinarene to maintain the structure of compound 1a in solution has been obtained via 1D and 2D ¹H NMR measurements. Indeed, the spectrum of of C-undecylcalix[4]resorcinarene in benzene-d₆ displays resonances attributed to the chiral and achiral calixarenes as well as the water molecules that make up compound 1a, as shown in Fig. 5. In particular the CH hydrogen atoms of the bridging methines, at 3.7 X 10⁻³ M, exhibit two broad singlets at 4.69 and 4.90 ppm (relative intensities 2:1) while their α-CH₂ hydrogens exhibit a broad singlet at 2.52 ppm and a pair of broad multiplets at 2.50 and 3.31 ppm (relative intensities 4:1:1). These splittings and line broadening effects are consistent with the presence of appreciably populated C₄ and C_{2V} conformers undergoing concerted hydrogen-bond tunneling motions in which the singlet at 4.90 ppm and the two multiplets at 2.50 and 3.31 ppm are assigned to the C₂ᵥ, conformer owing to the presence of chiral methane carbon atoms that induce de symmetrization of the methylene protons. That the multiplets are coupled with the singlet at 4.90 ppm, as well as with each other, was verified by COSY NMR data. Furthermore, a broad singlet that shifts downfield with increasing concentration and integrates to 12 protons is observed at 5.91 ppm and is assigned to the 12 protons of the water molecules that contribute to supramolecular assembly 1 a. This assignment is also supported by D₂O exchange experiments. Indeed, these observations, coupled with a recent molecular mass determination by vapor pressure osmometry in benzene (7066 g mol⁻¹),(Aoyama et al. J. Am. Chem. Soc. 111, 5397-5404 (1989)) provide convincing evidence supporting the ability of C-undecylcalix[4]resorcinarene to maintain the structure of compound 1 a in solution.

Our discovery that C-methylcalix[4]resorcinarene and of C-undecylcalix[4]resorcinarene form spherical molecular assemblies could bear relevance in a number of areas. In addition to providing new insight into design criteria for the construction of spherical hosts, that supramolecular assembly 1a sustains its cavity in both solution and the solid state suggests that supramolecular assembly 1a could be exploited to package guests within its interior. This could lead to a number of applications such as supramolecular assembly 1a as: (1)a chiral catalyst for chemical transformations, (2) a catalyst for chemical transformations, (3) a medium for chemical transformations, (4) materials with beneficial magnetic, optical, or electric properties, (5) a microvesicle for drug delivery to mammals,(Mathiowitz et al. Nature 386, 410-414 (1997)) and (6) an intermediate for separations problems. (Atwood et al. Nature, 368, 229-231 (1994)) Indeed, molecular modeling experiments with fragments obtained from the Cambridge Structural Database illustrate that the interior of supramolecular assembly 1a is spacious enough to accommodate relatively large substrates such as coordination compounds (e. g. MLₙ where n is an integer taking the values 1- 6), fullerenes (e. g. C₆₀), and supramolecular hosts (e.g. porphyrins). Considering the current commercial availability of C-methylcalix[4]resorcinarene and of C undecylcalix[4]resorcinarene, supramolecular assembly 1a may now be recognized as readily available spheroid in a burgeoning area of spherical host chemistry.

The following examples illustrate the invention

### EXAMPLE 1

Addition of C-methylcalix[4]resorcinarene (0.015 g) to a boiling aliquot of neat nitrobenzene (5 mL) followed by cooling to room temperature yielded light yellow cubic crystals suitable for X-ray analysis. The formulation of supramolecular assembly la was confirmed by single-crystal X-ray diffraction and ¹H NMR spectroscopy.

### EXAMPLE 2 (NOT CLAIMED)

Preparation of the cube. 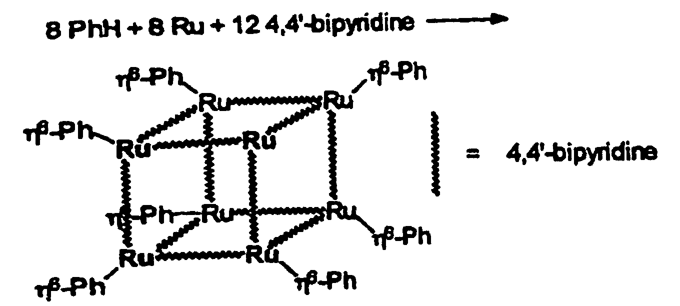 wherein the Ru atoms are linked together by the 4,4'-bipyridine synthons by coordinate covalent bonds.

### EXAMPLE 3 (NOT CLAIMED)

Preparation of the rhombicosidodecahedron. 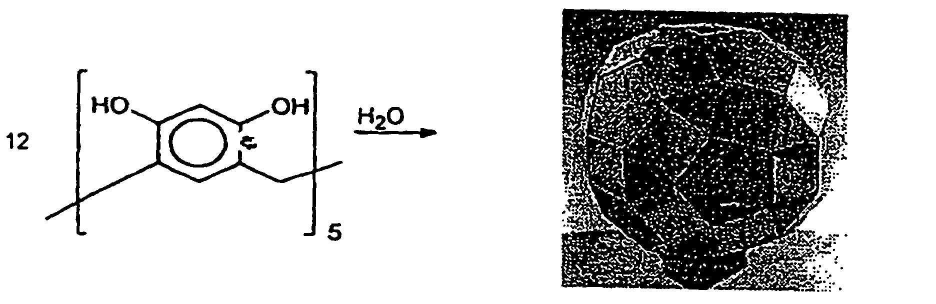 wherein the calix[5]resorcinarene synthons are bonded via 0-H... O hydrogen bonds from one of the said synthons to the other said synthons.

### EXAMPLE 4 (NOT CLAIMED)

Preparation of the rhombitruncated cuboctahedron. 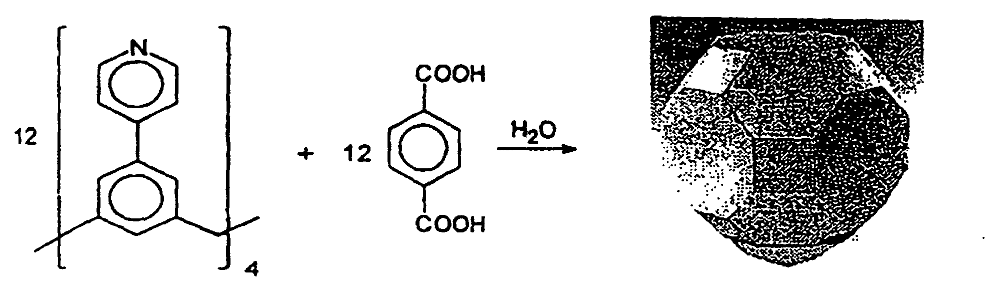 wherein a substituted calix[4]arene lies on each square face and the calixarenes are bridged by the 1,4-benzene dicarboxylic acid synthons. The hexagonal faces are formed from an edge from each of three calixarene synthons and three 1,4-benzene dicarboxylic acid synthons. The octagonal faces are formed from an edge from each of four calixarene synthons and four 1,4,-benzene dicarboxylic acid synthons.

### EXAMPLE 5 (NOT CLAIMED)

Preparation of the rhombicuboctahedron. 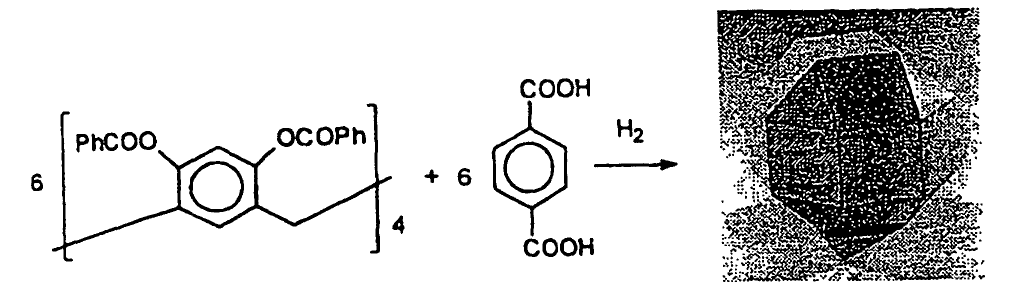 wherein the substituted calix[4]resorcinarenes lie on every other square face and said square faces are linked through hydrogen bonds by the 1,4-benzene dicarboxylic acid synthons.

A substantially spherical molecular or ionic synthon assembly has a substantially enclosed volume greater than 1300 A³ and comprises six calix[4]resorcinarenes and eight water molecules as the synthons, the assembly mimick a snub cube.

Advantageously the cyclic calix-type compound is a calixresorcinarene of the formula 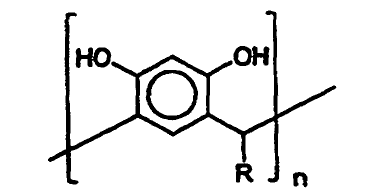 wherein n is an integer taking the values 4-8 and the R groups are independently selected from the group consisting of hydrocarbon, heterosubstituted hydrocarbon, and heteroaryl groups.

The substantially spherical supramolecular assembly having a substantially enclosed volume greater than 350 A³.

The substantially spherical molecular or ionic synthon assembly having a substantially enclosed volume greater than 1300 A³ and comprising six calix[4]resorcinarenes and eight water molecules as the synthons, the assembly mimicking a snub cube.

## Claims

1. A substantially spherical supramolecular assembly having a substantially enclosed volume and a framework structure that mimics a Platonic or Archimedean solid, the supramolecular assembly comprising more than four synthons held together by a combination of carbon-based covalent, non-covalent, and/or coordinate covalent bonds, with at least two of the bonds being selected from the group consisting of noncovalent bonds and coordinate covalent bonds, wherein the synthons comprise water molecules and calixresorcinarenes of the formula: 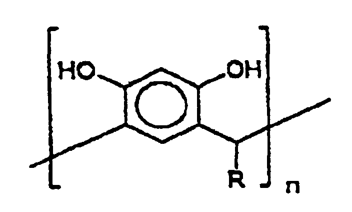 wherein n is an integer within the range of 4-8 and the R groups are independently selected from the group consisting of hydrocarbon, heterosubstituted hydrocarbon, and heteroaryl groups.

2. The substantially spherical supramolecular assembly according to claim 1 wherein said synthons comprise said water molecules and calixresorcinarenes in a ratio of eight water molecules to six calixresorcinarenes.

3. The substantially spherical supramolecular assembly according to claim 1 having a substantially enclosed volume greater than 350 A³.

4. The substantially spherical supramolecular assembly according to claim 1 wherein n is 4 to provide a calix[4]resorcinarene.

5. The substantially spherical supramolecular assembly according to claim 4 wherein said substantially spherical supramolecular assembly comprises six of said calix[4]resorcinarenes.

6. The substantially spherical supramolecular assembly according to claim 5 wherein said spherical supramolecular assembly comprises six C-methylcalix[4]resorcinarenes.

7. The substantially spherical supramolecular assembly according to claim 6 wherein said spherical supramolecular assembly comprises six C-undecylcalix[4]resorcinarenes.

8. A substantially spherical supramolecular assembly according to claim 4 comprising a hexamer of a calix[4]resorcinarene **characterized by** the formula: 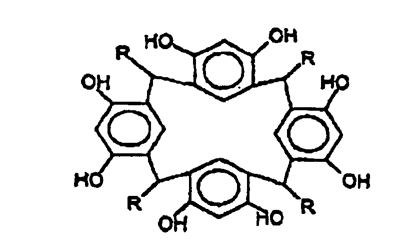 wherein R is a hydrocarbyl, heterosubstituted hydrocarbon, or heteroaryl group.

9. The substantially spherical supramolecular assembly according to claim 8 having an enclosed interior volume greater than 1300 A³ angstrums.

10. The substantially spherical supramolecular assembly according to claim 9 in which R is hydrogen.

11. The substantially spherical supramolecular assembly according to claim 9 in which R is methyl.

12. The substantially spherical supramolecular assembly according to claim 9 in which R is undecyl.

13. A substantially spherical supramolecular or ionic synthon assembly according to claim 9 having a substantially enclosed volume greater than 1300 A³ and comprising six calix[4]resorcinarenes and eight water moleculas as the synthons, the assembly mimicking a snub cube.

## Patentansprüche

1. Eine im wesentlichen sphärische supramolekulare Verbindung mit im wesentlichen geschlossenem Volumen und einer Rahmenstruktur, die einen platonischen oder archimedischen Körper nachahmt, wobei die supramolekulare Verbindung mehr als vier Synthone umfasst, zusammengehalten durch eine Kombination von kovalenten Verbindungen auf Kohlenstoffbasis, nichtkovalenten, und/oder koordinierten kovalenten Verbindungen, wobei mindestens zwei der Verbindungen aus der Gruppe bestehend aus nichtkovalenten Verbindungen und koordinierten kovalenten Verbindungen stammen, wobei die Synthone Wassermoleküle umfassen wie auch Calixresorcinarene mit der Formel: 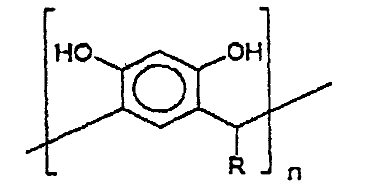 wobei n eine ganze Zahl innerhalb des Bereichs von 4-8 ist und die R-Gruppen unabhängig ausgewählt aus der Gruppe bestehend aus Kohlenwasserstoff, heterosubstituiertem Kohlenwasserstoff und Heteroarylgruppen sind.

2. Im wesentlichen sphärische supramolekulare Verbindung nach Anspruch 1 wobei die besagten Synthone besagte Wassermoleküle und Calixresorcinarene in einem Verhältnis von acht Wassermolekülen zu sechs Calixresorcinarenen enthalten.

3. Im wesentlichen sphärische supramolekulare Verbindung nach Anspruch 1 mit einem im wesentlichen geschlossenen Volumen größer als 350 A3.

4. Im wesentlichen sphärische supramolekulare Verbindung nach Anspruch 1 wobei n 4 ist, um ein Calix[4]resorcinaren zu bilden.

5. Im wesentlichen sphärische supramolekulare Verbindung nach Anspruch 4, wobei die im wesentlichen sphärische supramolekulare Verbindung sechs der besagten Calix[4]resorcinarene umfasst.

6. Im wesentlichen sphärische supramolekulare Verbindung nach Anspruch 5, wobei die sphärische supramolekulare Verbindung sechs C-methylcalix[4]resorcinarene umfasst.

7. Im wesentlichen sphärische supramolekulare Verbindung nach Anspruch 6, wobei die besagte sphärische supramolekulare Verbindung sechs C-undecylcalix[4]resorcinarene umfasst.

8. Im wesentlichen sphärische supramolekulare Verbindung nach Anspruch 4 mit einem Hexamer eines Calix[4]resorcinarens, **gekennzeichnet durch** die Formel: 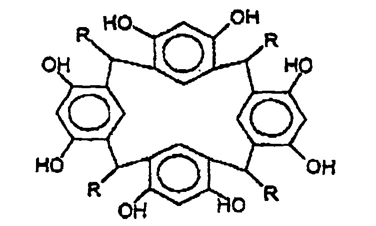 wobei R ein Hydrocarbyl, heterosubstituierter Kohlenwasserstoff oder eine Heteroarylgruppe ist.

9. Im wesentlichen sphärische supramolekulare Verbindung nach Anspruch 8 mit einem geschlossenen Innenvolumen größer als 1300 A3 Angström.

10. Im wesentlichen sphärische supramolekulare Verbindung nach Anspruch 9, bei der R Wasserstoff ist.

11. Im wesentlichen sphärische supramolekulare Verbindung nach Anspruch 9, bei der R Methyl ist.

12. Im wesentlichen sphärische supramolekulare Verbindung nach Anspruch 9 bei der R Undecyl ist.

13. Im wesentlichen sphärische supramolekulare oder ionische Synthonenverbindung nach Anspruch 9 mit im wesentlichen geschlossenem Volumen größer als 1300 A3 und mit sechs Calix[4]resorcinarenen und acht Wassermolekülen als Synthone, wobei die Verbindung einen schiefen Würfel imitiert.

## Revendications

1. Un composé supramoléculaire substantiellement sphérique ayant un volume substantiellement enfermé et une structure charpentée qui mime un solide Platonicien ou d'Archimède, le composé supramoléculaire comprenant plus de quatre synthons tenus ensemble par une combinaison de liaisons covalentes à base de carbone, non-covalentes, et/ou liaisons covalentes coordonnées, dont au moins deux étant sélectionnées du groupe composé de liaisons noncovalentes et liaisons covalentes coordonnées, les synthons comprenant des molécules d'eau et des calixrésorcinarènes de la formule: 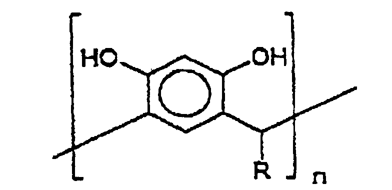 dans lequel n est un nombre entier dans la gamme de 4-8 et les groupes R sont indépendamment sélectionnés du groupe composé d'hydrocarbure, hydrocarbure hétérosubstitué, et groupes de hétéroaryle.

2. Le composé supramoléculaire substantiellement sphérique selon la revendication 1 dans lequel lesdits synthons comprennent lesdites molécules d'eau et calixrésorcinarènes dans une proportion de huit molécules d'eau à six calixrésorcinarènes.

3. Le composé supramoléculaire substantiellement sphérique selon la revendication 1 ayant un volume substantiellement enfermé supérieur à 350 A3.

4. Le composé supramoléculaire substantiellement sphérique selon la revendication 1 dans lequel n est 4 pour former un calix[4]résorcinarène.

5. Le composé supramoléculaire substantiellement sphérique selon la revendication 4 dans lequel ledit composé supramoléculaire substantiellement sphérique comprend six desdits calix[4]résorcinarènes.

6. Le composé supramoléculaire substantiellement sphérique selon la revendication 5 dans lequel ledit composé supramoléculaire sphérique comprend six C-methylcalix[4]résorcinarènes.

7. Le composé supramoléculaire substantiellement sphérique selon la revendication 6 dans lequel ledit composé supramoléculaire sphérique comprend six C-undecylcalix[4]résorcinarènes.

8. Un composé supramoléculaire substantiellement sphérique selon la revendication 4 comprenant un hexamère d'un calix[4]résorcinarène **caractérisé par** la formule: 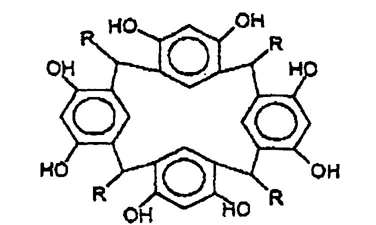 dans lequel R est un hydrocarbyle, hydrocarbure hétérosubstitué, ou groupe de hétéroaryles.

9. Le composé supramoléculaire substantiellement sphérique selon la revendication 8 ayant un volume intérieur enfermé supérieur à 1300 A3 angstrums.

10. Le composé supramoléculaire substantiellement sphérique selon la revendication 9 dans lequel R est hydrogène.

11. Le composé supramoléculaire substantiellement sphérique selon la revendication 9 dans lequel R est méthyle.

12. Le composé supramoléculaire substantiellement sphérique selon la revendication 9 dans lequel R est undécyle.

13. Un composé de synthons substantiellement sphérique supramoléculaire ou ionique selon la revendication 9 ayant un volume substantiellement enfermé supérieur à 1300 A3 et comprenant six calix[4]résorcinarènes et huit molécules d'eau en qualité de synthons, l'composé mimant un cube incliné.
